# EUROPÄISCHE PATENTSCHRIFT

(11) **EP 1 372 578 B1**
(45) Veröffentlichungstag und Bekanntmachung des Hinweises auf die Patenterteilung: **22.08.2007**
(21) Anmeldenummer: 01985669.9
(22) Anmeldetag: 28.09.2001
(51) Int. Cl.: A61Q 3/02, A61K 8/92

(54) **KOSMETISCHER ODER PHARMAZEUTISCHER NAGELLACK, NAGELLACK-BASIS ODER NAGELHÄRTER**
COSMETIC OR PHARMACEUTICAL NAIL POLISH, NAIL POLISH BASE OR NAIL HARDENER
VERNIS A ONGLES, BASE DE VERNIS A ONGLES, OU DURCISSEUR COSMETIQUE OU PHARMACEUTIQUE

(30) Priorität: 28.09.2000 DE 20018291 U
(43) Veröffentlichungstag der Anmeldung: 02.01.2004
(73) Patentinhaber: Wohnhas, Jerg, 59590 Geseke (DE)
(72) Erfinder: Wohnhas, Jerg, 59590 Geseke (DE)
(86) Internationale Anmeldenummer: PCT/EP2001/011268
(87) Internationale Veröffentlichungsnummer: WO 2002/026199

(56) Entgegenhaltungen:
- FR-A- 1 156 213
- FR-A- 1 521 072

## Beschreibung

Die Erfindung betrifft kosmetischen oder pharmazeutischen nagellack, nagellack-basis oder nagel harter,
dadurch gekennzeichnet
dass sie erfindungsgemäß ausschließlich aus pflanzlichen Rohstoffen (Benzoe-Öl und/oderMyrrhen-Öl), pflanzlich-tierischem Rohstoff (Schellack) bestehen und als Lösungsmittel nur ein pflanzlicher Alkohol (Äthylalkohol) in Kombination mit pflanzlichen ätherischen Ölen verwendet wird. Der Gesamtanteil von Benzoe-Öl, Myrrhen-Öl, Schellack und Äthanol beträgt mindestens 50 bis 97 %. In geringen Mengen bis zu 0,6 % lassen sich problemlos andere pflanzliche Öle (Rückfetter) und bis zu 50 % pflanzliche ätherische Öle (für therapeutische Zwecke oder als Duftkomponente) hinzugeben.

Wesentlicher Bestandteil dieser Rezeptur ist die Verwendung von Schellack. Weder Chemiker und Pharmazeuten noch Tierschützer, Vegetarier und Veganer konnten sich bis dato einigen, ob der Schellack pflanzlichen oder tierischen Ursprung hat. Definitiv stammt er nicht vom toten Tier und entspricht daher der vegetarischen Ethik. Ebenso wenig kann man ihn als rein pflanzlichen Rohstoff einstufen, wonach er nicht der veganen Ethik entspricht. Um diesem Streit aus dem Wege zu gehen bezeichnen wir hier den Ursprung des Schellacks als "pflanzlich-tierisch".
(siehe Anlage 1: Römpps Chemie-Lexikon Bd.4, 8. Auflage 1985, Seiten 3706-3707)

Mit der vorliegenden Erfindung lassen sich
- neutraler Bio-Nagellack und Bio-Nagellack-Basis (zur späteren Einfärbung) sowie
- Bio-Nagelhärter herstellen.
Der Unterschied liegt in der Konzentration und dem Verhältnis der eingesetzten Rohstoffe.

Der im Patentanspruch angegebenen Erfindung liegen folgende Probleme zu Grunde:
- Nagellacke enthalten eine Vielzahl von gesundheitsschädlichen, allergisierenden, sensibilisierenden und in der Herstellung umweltfeindlichen Rohstoffe wie zum Beispiel: Xylol, Toluol, Benzole, Ketone, PAKs, Nitrocellulose, Phtalate, Acetate, Formaldehyd, Kunstharze usw.

- Vereinzelt werden in Nagellacken in ganz geringen Anteilen auch Benzoeharz, Schellack und Äthanol eingesetzt. Diese werden aber ausschließlich in Verbindung mit anderen als den hier genannten Stoffen verwendet und werden vornehmlich zur Verbesserung von Eigenschaften (Glanz und Adhäsion) zugesetzt.
   (siehe Anlagen 2 bis 4:
- Römpps Chemie-Lexikon Bd.4, 8. Aufl. 1985, Seite 2707
- Hugo Janistyn, Handbuch der Kosmetika und Riechstoffe, Hüthig Verlag Heidelberg 1969, 2. Auflage, Seiten 934 -935
- G.A. Novak, Die kosmetischen Präparate, Verlag chem. Industrie, Band 2, 3. Auflage 1982, Seiten 683 bis 697, 705 bis 707).

- Ein weiterer Nachteil der bekannten Nagellacke besteht darin, dass diese nur mit herkömmlichen Nagellackentfernen, welche die gleiche Problematik aufweisen, entfernt werden können.

Ein Nagellack oder Nagelhärter lässt sich schon mit nur Schellack und Alkohol herstellen.

Dieser ist aber einerseits zu dünnflüssig und andererseits glanzlos. Optimal ist die Kombination mit Benzoe- und oder Myrrhen-Öl.
- Glanz und/ oder Verdickung können sonst nur mit chemischen Substanzen erzielt werden oder mit anderen nätürlichen Substanzen, welche aber die Trockungszeit verlängern, eine schlechte Oberflächentrockung verursachen, einen unangenehmen Geruch oder Geschmack haben oder zu einer unschönen Lackoberfläche (Orangenhauteffekt) oder Weißeln führen.

Die im Patentanspruch angegebene Erfindung kommt dem Wunsch der Verbraucher, Hautärzte, Allergologen usw. nach einem Natur-Nagellack ohne schädliche Substanzen und mit überschaubaren und lang-bekannten guten und unbedenklichen-Rohstoffen-entgegen..

Die mit der Erfindung erzielten Vorteile bestehen darin, dass trotz Verzicht auf die genannten üblichen Rohstoffe mit der beschriebenen Rohstoff-Kombination ein Produkt hergestellt werden kann, das den Ansprüchen "schnelle Trocknungszeit, Glanz, Härte und Widerstandskraft" in ausreichendem Maße gerecht wird und darüber hinaus durch die Mikroverkapselung einen lang anhaltenden "angenehmen Eigengeruch" besitzt. Ein weiterer Vorteil liegt darin, dass dieser Nagellack schon mit 70%-igem Äthylalkohol-anstelle von herkömmlichen chemischen Nagellackentfernern entfernt werden kann.

Der Einsatz der Erfindung als Nagellack oder Nagelhärter ist abhängig von der Konzentration der Alkohol-Schellack-Mischung und dem eingesetzten Benzoe-Öl.

Die Erfindung läßt sich wie oben genannt auch als Basis für farbliche Nagellacke einsetzen.

### Die Rezepturen und die Herstellung der Produkte

Die Art der Konzentration des Schellacks und das Verhältnis zum Benzoe-Öl und/ oder einem anderen Baumharz richtet sich nach dem Anwendungszweck und den Anforderungen an das Endprodukt.
Dementsprechend können die nachfolgenden Rezepturbeispiele nur einen groben Überblick geben. Die Art des verwendeten Schellacks, die Kombination mit dem Äthylalkohol und dem Benzoe-Öl oder anderen ätherischen Ölen (insbesondere im Rahmen der Aromatherapie) können variieren.

Als Äthylalkohol werden folgende unvergällten Alkoholtypen und Mischungen verwendet:
Alkohol Typ 410 oder 430 vermischt mit 0,5 % bis 1,0 % echtem Japankampfer, Lavendelöl, Citronellöl , Teebaumöl oder einer Mischung der genannten ätherischen Öle.
Als Benzoe-Öl (1:1) wird wegen der Fließfähigkeit eine Mischung aus 50 % Benzoe-Öl und 50 % des o.g. Alkoholgemisches eingesetzt.

Als Schellack wird entwachster Blätterschellack - möglichst sprühgetrocknet - in unterschiedlichen Färbungs- und Bleichungsgraden eingesetzt.

### Beispiel 1

Nagellack Seidenglanz Qualität hoch

| | |
|---|---|
| Herstellungsmenge: | 1.000 g |
| Benötigte Geräte: | Propellerrührer 900 U/min |
| | Wärmebad 40 Grad C |

| | | | |
|---|---|---|---|
| Rezeptur: | A Schellack-Alkohol-Phase | Schellack sprühgetrocknet | 120,0 g |
| | | Alkohol 96 % | 650,0 g |
| | | | |
| | B Zusatz-Phase | Benzoe-Öl 1:1 | 230,0 g |

Der Schellack wird bei 40 Grad C mit dem Propellerrührer in dem Alkohol gelöst. Nach vollständiger Lösung wird das Benzoe-Öl untergemischt.

### Beispiel 2

Nagellack Seidenglanz Qualität mittel

| | |
|---|---|
| Herstellungsmenge : | 1.000 g |
| Benötigte Geräte: | Propellerrührer 900 U/min |
| | Wärmebad 40 Grad C |

| | | | |
|---|---|---|---|
| Rezeptur: | A Schellack-Alkohol-Phase | Schellack sprühgetrocknet | 90,0 g |
| | | Alkohol 96 % | 760,0 g |
| | | | |
| | B Zusatz-Phase | Benzoe-Öl 1:1 | 150,0 g |

Der Schellack wird bei 40 Grad C mit dem Propellerrührer in dem Alkohol gelöst. Nach vollständiger Lösung wird das Benzoe-Öl untergemischt.

### Beispiel 3

Nagelhärter

| | |
|---|---|
| Herstellungsmenge: | 1.000 g |
| Benötigte Geräte: | Propellerrührer 900 U/min |
| | Wärmebad 35 Grad C |

| | | | |
|---|---|---|---|
| Rezeptur: | A Schellack-Alkohol-Phase | Schellack sprühgetrocknet | 60,0 g |
| | | Alkohol 96 % | 922,0 g |
| | | | |
| | B Zusatz-Phase | Benzoe-Öl 1:1 | 15,0 g |
| | | Myrrhen-Öl | 3,0 g |

Der Schellack wird bei 40 Grad C mit dem Propellerrührer in dem Alkohol gelöst. Nach vollständiger Lösung wird das Benzoe-Öl untergemischt.

### Beispiel 4

Aromatherapeutischer Nagelhärter zur Fußpilz-Prophylaxe

| | |
|---|---|
| Herstellungsmenge: | 1.000 g |
| Benötigte Geräte: | Propellerrührer 900 U/min |
| | Wärmebad 35 Grad C |

| | | | |
|---|---|---|---|
| Rezeptur: | A Schellack-Alkohol-Phase | Schellack sprühgetrocknet | 60,0 g |
| | | Alkohol 96 % | 873,0 g |
| | | | |
| | B Zusatz-Phase | Benzoe-Öl 1:1 | 30,0 g |
| | | Teebaumöl | 18,0 g |
| | | Pfefferminzöl China | 15,0 g |
| | | Thymianöl | 4,0 g |

Der Schellack wird bei 40 Grad C mit dem Propellerrührer in dem Alkohol gelöst. Nach vollständiger Lösung wird das Benzoe-Öl mit den ätherischen Ölen untergemischt.

### Beispiel 5

Nagelhärter einfach

| | |
|---|---|
| Herstellungsmenge: | 1.000 g |
| Benötigte Geräte: | Propellerrührer 900 U/min |
| | Wärmebad 35 Grad C |

| | | | |
|---|---|---|---|
| Rezeptur: | A Schellack-Alkohol-Phase | Schellack sprühgetrocknet | 50,0 g |
| | | Alkohol 96 % | 949,0 g |
| | | | |
| | B Zusatz-Phase | Benzoe-Öl 1:1 | 1,0 g |

Der Schellack wird bei 40 Grad C mit dem Propellerrührer in dem Alkohol gelöst. Nach vollständiger Lösung wird das Myrrhen-Öl untergemischt.

## Patentansprüche

1. Kosmetischer oder pharmazeutischer farbloser Nagellack, Nagellack-Basis, oder Nagelhärter, der **dadurch gekennzeichnet ist, dass** er zu mindestens 50 % aus nur reinem Alkohol, Schellack und Benzoe-Öl besteht.

2. Kosmetischer oder pharmazeutischer farbloser Nagellack, Nagellack-Basis oder Nagelhärter gemäß vorstehendem Anspruch, **dadurch gekennzeichnet, dass** er zu mindestens 80 % aus nur reinem Alkohol, Schellack und Benzoe-Öl besteht.

3. Kosmetischer oder pharmazeutischer farbloser Nagellack, Nagellack-Basis oder Nagelhärter gemäß vorstehendem Anspruch, **dadurch gekennzeichnet, dass** er zu mindestens 90 % aus nur reinem Alkohol, Schellack und Benzoe-Öl besteht.

4. Kosmetischer oder pharmazeutischer farbloser Nagellack, Nagellack-Basis oder Nagelhärter gemäß vorstehendem Anspruch, **dadurch gekennzeichnet, dass** er zu mindestens 97 % aus nur reinem Alkohol, Schellack und Benzoe-Öl besteht.

## Claims

1. Cosmetical or pharmaceutical colourless nail varnish, nail varnish base or nail hardener **characterized by** it's consisting only of pure alcohol, shellac, and benzoin oil per at least 50%.

2. Cosmetical or pharmaceutical colourless nail varnish, nail varnish base or nail hardener **characterized by** it's consisting according to the above claim only of pure alcohol, shellac, and benzoin oil per at least 80 %.

3. Cosmetical or pharmaceutical colourless nail varnish, nail varnish base or nail hardener **characterized by** it's consisting according to the above claim only of pure alcohol, shellac, and benzoin oil per at least 90 %.

4. Cosmetical or pharmaceutical colourless nail varnish, nail varnish base or nail hardener **characterized by** it's consisting according to the above claim only of pure alcohol, shellac, and benzoin oil per at least 97 %.

## Revendications

1. Vernis à ongles incolore cosmétique ou pharmaceutique, avec base de vernis à ongles ou de durcisseur pour ongles **caractérisée par le fait qu'**elle ne consiste qu'en alcool pur, gomme-laque, et huile de benzoïne, à 50% au moins.

2. Vernis à ongles incolore cosmétique ou pharmaceutique, avec base de vernis à ongles ou de durcisseur pour ongles **caractérisée par** le fait, selon l'affirmation reportée ci-dessus, qu'elle ne consiste qu'en alcool pur, gomme-laque, et huile de benzoïne, à 80% au moins.

3. Vernis à ongles incolore cosmétique ou pharmaceutique, avec base de vernis à ongles ou de durcisseur pour ongles **caractérisée par** le fait, selon l'affirmation reportée ci-dessus, qu'elle ne consiste qu'en alcool pur, gomme-laque, et huile de benzoïne, à 90% au moins.

4. Vernis à ongles incolore cosmétique ou pharmaceutique, avec base de vernis à ongles ou de durcisseur pour ongles **caractérisée par** le fait, selon l'affirmation reportée ci-dessus, qu'elle ne consiste qu'en alcool pur, gomme-laque, et huile de benzoïne, à 97% au moins%.
